# EUROPEAN PATENT SPECIFICATION

(11) **EP 3 472 353 B1**
(45) Date of publication and mention of the grant of the patent: **04.08.2021**
(21) Application number: 17754192.7
(22) Date of filing: 15.06.2017
(51) Int. Cl.: C12Q 1/689

(54) **METHOD OF DETECTING SALMONELLA TYPHIMURIUM**
VERFAHREN ZUM NACHWEIS VON SALMONELLA TYPHIMURIUM
PROCÉDÉ DE DÉTECTION DE SALMONELLA TYPHIMURIUM

(30) Priority: 16.06.2016 US 201662351130 P
(43) Date of publication of application: 24.04.2019
(73) Proprietor: Bio-Rad Laboratories, Inc., Hercules, CA 94547 (US)
(72) Inventor: MOUSCADET, Jean-François, Hercules, CA 94547-1803 (US); PIERRE, Sophie, Hercules, CA 94547-1803 (US)
(74) Representative: dompatent von Kreisler Selting Werner - Partnerschaft von Patent- und Rechtsanwälten mbB
(86) International application number: PCT/IB2017/000921
(87) International publication number: WO 2017/216635

(56) References cited:
- WO-A1-95/00664
- CN-A- 101 705 307
- CN-A- 104 087 654
- CN-A- 104 830 988
- H J KIM ET AL: "Identification of Salmonella enterica serovar Typhimurium using specific PCR primers obtained by comparative genomics in Salmonella serovars.", J FOOD PROT., vol. 69, no. 7, 1 July 2006 (2006-07-01), pages 1653-1661, XP055408489,

### SEQUENCE LISTING

<110> Bio-Rad Laboratories, Inc. Mouscadet, Jean-Francois Pierre, Sophie
<120> METHOD OF DETECTING SALMONELLA TYPHIMURIUM
<130> 1050901
<150> US 62/351,130
   <151> 2016-06-16
<160> 9
<170> PatentIn version 3.5
<210> 1
   <211> 1388
   <212> DNA
   <213> Salmonella typhimurium
<400> 1
   atgtagctta agatatctat agtgatatca gtgtaatact tattggttag atcggtatga      60
   tcttgaatat ttttatatcg atagtttgga ttacatagta gagttatttc actttgcaat     120
   acagctttaa ttatagtttt gtcaagttgt aatttatcta taaaaatatt atttatagta     180
   ttttctatta ggagaagtgt ttcgactaac ttgatatttg tattgatttt ttgtttgtag     240
   atattccgta gcaattgagt tgaattgtgt tcaagcaatg gtgaacaaac ataatcccat     300
   gattgctctt gagagtccca gtcattttta gctatttcaa tagcattggt gactaattcg     360
   ataatttcat cttcaatttc tggatatggt actgaggcta attcaccact agtaaagcta     420
   agtgtggggg ctagtattga taaataatgg tttacaaccg gagtgcacat taatcccgca     480
   gcgtaaagca actcattttt gttatttgaa aagccgcaac ggcctgtatc atcaaaaaca     540
   aacccttttg gacgatatct cacgcaaaaa ttaccttggc ttatttttga ccatgttatg     600
   ccttctctaa agtaatactc atcatttctt acggcagagc gagttttgcc attctcaaat     660
   ttaaaatttc gtatttcgta accattattt tcccaattta caactatttc gttattacca     720
   taccactttc gatattcacc tccactacta caaggaaacc atttgatatt atgaatgtcg     780
   atttttgtat ttgattcttt atttgtgata agggtttttt ttattgaaac ctcgtaccaa     840
   tatctttgaa atttaatatt gtcaccggtg gacatgcctg cttttaatgc tattttttct     900
   ccaagttttt tatggtggcg aaaagataat agactcggta agtctatcca atatgctatt     960
   ggcattcctg gtatgttttt aaaatcatgc tgtgtaaatt tatcaaatat atttttcctt    1020
   agaagtagat cgcttttctt tacttcttcc ctaccatcta taagtctaaa aaatacaggt    1080
   tggtaacgtt cggagtgttg gtttttaatc acccaggcag ttgtctgtac aacctctcca    1140
   gaaatttgcc caaaagcccg agctcccaaa tgtgccatcg taataaatgt tttattgtcc    1200
   aataaccagt tacgtagtgc ttcataactt gacaaaaaca tccatgattg catattgact    1260
   tgagcattaa acccattttc tttaagcaaa gaaaatgcat tctgcataaa cattgcaaac    1320
   aaatcagctt tactatccgg gaagttattt ttggcaaact ctttcagctc actattcatt    1380
   cccttgcc                                                             1388
<210> 2
   <211> 947
   <212> DNA
   <213> Salmonella typhimurium
<400> 2
   atgtagctta agatatctat agtgatatca gtgtaatact tattggttag atcggtatga      60
   tcttgaatat ttttatatcg atagtttgga ttacatagta gagttatttc actttgcaat     120
   acagctttaa ttatagtttt gtcaagttgt aatttatcta taaaaatatt atttatagta     180
   ttttctatta ggagaagtgt ttcgactaac ttgatatttg tattgatttt ttgtttgtag     240
   atattccgta gcaattgagt tgaattgtgt tcaagcaatg gtgaacaaac ataatcccat     300
   gattgctctt gagagtccca gtcattttta gctatttcaa tagcattggt gactaattcg     360
   ataatttcat cttcaatttc tggatatggt actgaggcta attcaccact agtaaagcta     420
   agtgtggggg ctagtattga taaataatgg tttacaaccg gagtgcacat taatcccgca     480
   gcgtaaagca actcattttt gttatttgaa aagccgcaac ggcctgtatc atcaaaaaca     540
   aacccttttg gacgatatct cacgcaaaaa ttaccttggc ttatttttga ccatgttatg     600
   ccttctctaa agtaatactc atcatttctt acggcagagc gagttttgcc attctcaaat     660
   ttaaaatttc gtatttcgta accattattt tcccaattta caactatttc gttattacca     720
   taccactttc gatattcacc tccactacta caaggaaacc atttgatatt atgaatgtcg     780
   atttttgtat ttgattcttt atttgtgata agggtttttt ttattgaaac ctcgtaccaa     840
   tatctttgaa atttaatatt gtcaccggtg gacatgcctg cttttaatgc tattttttct     900
   ccaagttttt tatggtggcg aaaagataat agactcggta agtctat                   947
<210> 3
   <211> 123
   <212> DNA
   <213> Salmonella typhimurium
<400> 3
   taggagaagt gtttcgacta acttgatatt tgtattgatt ttttgtttgt agatattccg      60
   tagcaattga gttgaattgt gttcaagcaa tggtgaacaa acataatccc atgattgctc     120
   ttg                                                                   123
<210> 4
   <211> 22
   <212> DNA
   <213> Artificial Sequence
<220>
   <223> synthetic forward primer
<400> 4
   taggagaagt gtttcgacta ac 22
<210> 5
   <211> 22
   <212> DNA
   <213> Artificial Sequence
<220>
   <223> synthetic reverse primer
<400> 5
   caagagcaat catgggatta tg 22
<210> 6
   <211> 29
   <212> DNA
   <213> Artificial Sequence
<220>
   <223> synthetic probe
<400> 6
   tttacaattg agttgaattg tgttcaagc 29
<210> 7
   <211> 30
   <212> DNA
   <213> Artificial Sequenc
<220>
   <223> synthetic probe
<400> 7
   aaaagaacac aattcaactc aattgctacg 30
<210> 8
   <211> 25
   <212> DNA
   <213> Artificial Sequence
<220>
   <223> synthetic probe
<400> 8
   caattgagtt gaattgtgtt caagc 25
<210> 9
   <211> 26
   <212> DNA
   <213> Artificial Sequence
<220>
   <223> synthetic probe
<400> 9
   gaacacaatt caactcaatt gctacg 26

## Description

### BACKGROUND

Salmonella is a leading cause of foodborne illnesses worldwide, with poultry and pork products being a primary source of infection to humans. Detecting Salmonella can be challenging because low levels of the bacteria may not be recovered using traditional culturing techniques. The genus Salmonella, member of the Enterobacteriaceae family, comprises two species *Salmonella enterica* and *Salmonella bongori. Salmonella enterica* is further divided into six subspecies, of which *S. enterica subsp. enterica* is the most clinically significant, causing 99% of Salmonella infections. The subspecies are further sub-divided into more than 2,500 serovars defined by somatic and flagellar antigens. *Salmonella enterica subsp. enterica* serovar Typhimurium and *Salmonella enterica subsp. enterica* serovar Enteritidis are the most frequently reported serovars associated with human cases of Salmonella infection from foodborne outbreaks. In the EU, a regulation in force since 2003 governs the mandatory detection of Salmonella. In 2011, this regulation was supplemented with the mandatory testing for S. Enteritidis and S. Typhimurium. According to Commission Regulation (EU) No. 1086/2011, all fresh poultry must be examined for S. Enteritidis and S. Typhimurium contamination. In the United States, the Food and Drug Administration (FDA) has published the Final Rule "Prevention of Salmonella Enteritidis in Shell Eggs During Production, Storage, and Transportation" (74 FR 33030), which will introduce methods requiring egg producers to test for S. Enteritidis. For non-egg producers, the FDA also published the guidance document for testing of human foods for salmonella: "Guidance for Industry: Testing for Salmonella Species in Human Foods and Direct-Human-Contact Animal Foods".

Conventional microbiological methods for the detection and identification of Salmonella serovars are very time consuming. The current accepted method for isolation of Salmonella from food and environmental primary production samples takes up to 5 days according to the ISO 6579. The most widely-used method used to characterize Salmonella into its subspecies is the Kauffman-White serotyping system, based on the variability of the O, H and Vi antigens. CN 101 705 307 A, CN 104 830 988 A and CN 104 087 654 A disclose PCR and LAMP methods of detecting *S. typhimurium.*

### SUMMARY

Described herein are methods and compositions for detecting *Salmonella* Typhimurium.

In an embodiment, a method of selectively detecting the presence of *Salmonella* Typhimurium in a sample comprises (a) providing a reaction mixture comprising a suitable primer pair for amplification of a sequence having at least 95% sequence identity over the entire length of the sequence of residues 755 to 1063 (309bp) of *Salmonella* Typhimurium ACCESSION CP007235 (SEQ ID NO:3); (b) performing PCR amplification of the nucleic acids of the sample using the reaction mixture of step (a) to amplify the sequence; and (c) selectively detecting the presence of *Salmonella* Typhimurium by detecting the amplified nucleic acids. In some embodiments, the step (b) is performed in partitions. In some embodiments, the detecting the presence of *Salmonella* Typhimurium comprises sequencing the amplified sequence.

In some embodiments, the reaction mixture comprises a primer pair for amplification of a sequence having at least, 97% or 99% identity over the entire length of the sequence SEQ ID NO:3 or a portion thereof.

In an embodiment, the primer pair for amplification of the nucleic acid region of SEQ ID NO:3 comprises the polynucleotide sequences set forth in SEQ ID NO:4 and SEQ ID NO:5. In an embodiment, the reaction mixture further comprises a probe for the nucleic acid region to be detected. In some embodiments, the probe comprises a detectable label. In some embodiment, the probe comprises the polynucleotide sequences set forth in SEQ ID NO:6 and SEQ ID NO:7. In certain embodiments, the probe comprises the polynucleotide sequences set forth in SEQ ID NO:6, SEQ ID NO:7, SEQ ID NO:8, or SEQ ID NO:9.

In an embodiment, an isolated polynucleotide comprises a polynucleotide sequence having at least 95% sequence identity, based on the BLASTN method of alignment over the entire length of the polynucleotide sequence set forth in SEQ ID NO:1. In some embodiments, the isolated polynucleotide sequence consists of the polynucleotide sequence set forth in SEQ ID NO:3.

In an embodiment, a kit for the detection of *Salmonella* Typhimurium in a sample comprises a primer pair comprising SEQ ID NO:4 and SEQ ID NO:5. In some embodiments, the kit further comprises a probe comprising SEQ ID NO:6 and SEQ ID NO:7. In some embodiments, the kit further comprises a probe comprising comprising SEQ ID NO:6, SEQ ID NO:7, SEQ ID NO:8, or SEQ ID NO:9. In certain embodiments, the kit further comprises at least one component selected from a lysis reagent, a DNA polymerase, at least one dNTP, a buffer, a negative control, a positive control, and instructions for performing a method to detect the presence of *Salmonella* Typhimurium in a nucleic acid sample.

### DETAILED DESCRIPTION

Provided herein are methods of selectively detecting the presence of *Salmonella* Typhimurium in a sample. Also provided are compositions for use in the detection of *Salmonella* Typhimurium in a sample by nucleic acid amplification, e.g., by real-time PCR.

The disclosed detection method finds utility in the detection of S. Typhimurium in any type of sample, for example in samples for food testing, environmental testing, or human/animal diagnostic testing. Exemplary food samples include, but are not limited to, meats products , poultry(e.g., chicken, turkey), eggs, fish (e.g, cod), cookie dough, produce (e.g, lettuce, tomatoes), dairy (e.g, cheese, milk), milk powder (e.g., infant formula), chocolate (e.g., milk), cocoa, nacho cheese seasoning, pasta, pet food, peanut butter, soy flour, spices, and ready-to-eat food. Environmental samples include, but are not limited to, plastic, sealed concrete, and stainless steel. Other types of samples include, but are not limited to, water, stool, blood, urine, and tissue. Another type of sample includes weeds. The methods may be performed at the farm or processing facility prior to initial packaging, after packaging (e.g., prior to or after export from one country to another), or at the point of sale.

### I. Definitions

Unless defined otherwise, technical and scientific terms used herein have the same meaning as commonly understood by a person of ordinary skill in the art. See, *e.g.,* Lackie, DICTIONARY OF CELL AND MOLECULAR BIOLOGY, Elsevier (4th ed. 2007); Green et al., MOLECULAR CLONING, A LABORATORY MANUAL (FOURTH EDITION), Cold Spring Harbor Laboratory Press (Cold Spring Harbor, NY 2012).

The term "a" or "an" is intended to mean "one or more." The term "comprise" and variations thereof such as "comprises" and "comprising," when preceding the recitation of a step or an element, are intended to mean that the addition of further steps or elements is optional and not excluded. The following definitions are provided to facilitate understanding of certain terms used frequently herein and are not meant to limit the scope of the present disclosure.

The term "nucleic acid" refers to polymers of deoxyribonucleotides or ribonucleotides in either single- or double-stranded form, and complements thereof. The term "polynucleotide" refers to a linear sequence of nucleotides. Nucleotides can be ribonucleotides, deoxyribonucleotides, or modified versions thereof. Examples of polynucleotides contemplated herein include single and double stranded DNA, single and double stranded RNA (including siRNA), and hybrid molecules having mixtures of single and double stranded DNA and RNA.

"Polymerase chain reaction" is abbreviated PCR.

The term "isolated" refers to materials, such as nucleic acid molecules and/or proteins, which are substantially free or otherwise removed from components that normally accompany or interact with the materials in a naturally occurring environment. Isolated polynucleotides can be purified from a host cell in which they naturally occur. Conventional nucleic acid purification methods known to skilled artisans can be used to obtain isolated polynucleotides. The term also embraces recombinant polynucleotides and chemically synthesized polynucleotides.

The terms "polynucleotide", "polynucleotide sequence", "nucleic acid sequence", and "nucleic acid fragment" are used interchangeably herein. These terms encompass nucleotide sequences and the like. A polynucleotide can be a polymer of RNA or DNA that is single- or double-stranded, that optionally contains synthetic, non-natural, or altered nucleotide bases. A polynucleotide in the form of a polymer of DNA can be comprised of one or more strands of cDNA, genomic DNA, synthetic DNA, or mixtures thereof.

The term "amplification product" refers to nucleic acid fragments produced during a primer-directed amplification reaction. Typical methods of primer-directed amplification include polymerase chain reaction (PCR), ligase chain reaction (LCR), or strand displacement amplification (SDA). If PCR methodology is selected, the replication composition can comprise the components for nucleic acid replication, for example: nucleotide triphosphates, two (or more) primers with appropriate sequences, thermostable polymerase, buffers, solutes, and proteins.

The term "primer" refers to a synthetic oligonucleotide that is capable of acting as a point of initiation of nucleic acid synthesis or replication along a complementary strand when placed under conditions in which synthesis of a complementary strand is catalyzed by a polymerase. A primer can further contain a detectable label, for example a 5' end label.

The term "probe" refers to a synthetic oligonucleotide that is complementary (though not necessarily fully complementary) to a polynucleotide of interest and forms a duplexed structure by hybridization with at least one strand of the polynucleotide of interest. A probe can further contain a detectable label.

As used herein, the terms "label", "detectable label", and such refer to a molecule capable of detection, including, but not limited to, radioactive isotopes, fluorescers, chemiluminescers, enzymes, enzyme substrates, enzyme cofactors, enzyme inhibitors, chromophores, dyes, metal ions, metal sols, semiconductor nanocrystals, and ligands (e.g., biotin, avidin, streptavidin, or haptens). A detectable label can also include a combination of a reporter and a quencher.

The term "reporter" refers to a substance or a portion thereof which is capable of exhibiting a detectable signal, which signal can be suppressed by a quencher. The detectable signal of the reporter is, e.g., fluorescence in the detectable range; thus, a reporter can also be a label.

The term "quencher" refers to a substance which is capable of suppressing, reducing, inhibiting, etc., the detectable signal produced by the reporter.

As used herein, the term "quenching" refers to a process whereby, when a reporter and a quencher are in close proximity, and the reporter is excited by an energy source, a substantial portion of the energy of the excited state non-radiatively transfers to the quencher where it either dissipates nonradiatively or is emitted at a different emission wavelength than that of the reporter (e.g., by fluorescence resonance energy transfer or FRET).

The reporter can be selected from fluorescent organic dyes modified with a suitable linking group for attachment to the oligonucleotide, such as to the terminal 3' carbon or terminal 5' carbon. The quencher can also be selected from organic dyes, which may or may not be fluorescent, depending on the embodiment of the invention. Generally, whether the quencher is fluorescent or simply releases the transferred energy from the reporter by non-radiative decay, the absorption band of the quencher should at least substantially overlap the fluorescent emission band of the reporter to optimize the quenching.

Non-fluorescent quenchers or dark quenchers typically function by absorbing energy from excited reporters, but do not release the energy radiatively.

Selection of appropriate reporter-quencher pairs for particular probes can be undertaken in accordance with known techniques. Fluorescent and dark quenchers and their relevant optical properties from which exemplary reporter-quencher pairs can be selected are listed and described, for example, in R. W. Sabnis, HANDBOOK OF FLUORESCENT DYES AND PROBES, John Wiley and Sons, New Jersey, 2015.

Reporter-quencher pairs can be selected from xanthene dyes including fluoresceins and rhodamine dyes. Many suitable forms of these compounds are available commercially with substituents on the phenyl groups, which can be used as the site for bonding or as the bonding functionality for attachment to an oligonucleotide. Another group of fluorescent compounds for use as reporters are the naphthylamines, having an amino group in the alpha or beta position. Included among such naphthylamino compounds are 1-dimethylaminonaphthyl-5 sulfonate, 1-anilino-8-naphthalene sulfonate and 2-p-touidinyl-6-naphthalene sulfonate. Other dyes include 3-phenyl-7-isocyanatocoumarin; acridines such as 9-isothiocyanatoacridine; N-(p-(2-benzoxazolyl)phenyl)maleimide; benzoxadiazoles; stilbenes; and pyrenes.

Suitable examples of quenchers can be selected from 6-carboxy-tetramethyl-rhodamine, 4-(4-dimethylaminophenylazo) benzoic acid (DABCYL), tetramethylrhodamine (TAMRA), BHQ-OTM, BHQ-1 TM, BHQ-2TM, and BHQ-3TM, each of which are available from Biosearch Technologies, Inc. of Novato, Calif., Qy7TM QSY-9TM, QSY-21 TM and QSY-35TM, each of which are available from Molecular Probes, Inc, Iowa Black™ FQ available from Integrated DNA Technologies.

Suitable examples of reporters can be selected from dyes such as SYBR green, 5-carboxyfluorescein (5-FAMTm available from Applied Biosystems of Foster City, Calif.), 6-carboxyfluorescein (6-FAM), tetrachloro-6-carboxyfluorescein (TET), 2,7-dimethoxy-4,5-dichloro-6-carboxyfluorescein, hexachloro-6-carboxyfluorescein (HEX), 6-carboxy-2',4,7,7'-tetrachlorofluorescein (6-TETTm available from Applied Biosystems), carboxy-X-rhodamine (ROX), 6-carboxy-4',5'-dichloro-2',7'-dimethoxyfluorescein (6-JOETM available from Applied Biosystems), VICTM dye products available from Molecular Probes, Inc., NEDTM dye products available from Applied Biosystems, Cal Fluor dye products (such as, e.g., Cal Fluor Gold 540, Orange 560, Red 590, Red 610, Red 635) available from Biosearch Technologies, Quasar dye products (such as, e.g., Quasar 570, 670, 705) available from Biosearch Technologies.

The term "percent identity," in the context of two or more nucleic acids, refers to two or more sequences or subsequences that are the same or have a specified percentage of nucleotides that are the same or have at least 95%, 96%, 97%, 98%, 99%, or higher identity over a specified region, when compared and aligned for maximum correspondence over a comparison window or designated region) as measured using a BLAST or BLAST 2.0 sequence comparison algorithms with default parameters, or by manual alignment and visual inspection *(see e.g.,* the NCBI web site at ncbi.nlm.nih.gov/BLAST). Such sequences are then said to be "substantially identical." Percent identity is typically determined over optimally aligned sequences, so that the definition applies to sequences that have deletions and/or additions, as well as those that have substitutions. The algorithms commonly used in the art account for gaps. Typically, identity exists over a region comprising a sequence that is at least about 25 nucleotides in length, or over a region that is 50-100 nucleotides in length, or over the entire length of the reference sequence.

The terms "selectively" or "selective" with respect to nucleic acids refers to the discrimination between the target nucleic acid sequence (e.g., target sequence of *Salmonella* Typhimurium) over the non-target nucleic acid sequences (e.g., non-target sequence *Salmonella* Typhimurium). An assay is selective for a sequence if little or no hybridization of the primer or probe occurs with non-target sequence.

The terms "partitioning" or "partitioned" refer to separating an aqueous solution having one or more of a sample and reactant into a plurality of portions, or "partitions." Partitions can be solid or fluid. In some embodiments, a partition is a solid partition, *e.g*., a microchannel. In some embodiments, a partition is a fluid partition, *e.g*., a droplet. In some embodiments, a fluid partition *(e.g.,* a droplet) is a mixture of immiscible fluids *(e.g.,* water and oil). In some embodiments, a fluid partition (*e.g*., a droplet) is an aqueous droplet that is surrounded by an immiscible carrier fluid *(e.g.,* oil).

### II. Nucleic Acids

### Genome Detection Regions

A detection method is provided herein that is based on the identification of residues 755 to 1063 (309bp) of *Salmonella* Typhimurium ACCESSION CP007235 (see SEQ ID NO: 3 in Table 1).

**Table 1: Sequences**

| SEQ ID NO | DESCRIPTION | SEQUENCE |
|---|---|---|
| 1 | 1388 bp fragment from the 3315 bp gene of *Salmonella* Typhimurium ACCESSION CP007235 | |
| | | |
| 2 | 947 bp fragment of *Salmonella* Typhimurium ACCESSION CP007235 | |
| 3 | 123 bp fragment of *Salmonella* Typhimurium ACCESSION CP007235 | |
| 4 | Forward Primer for 123 bp target sequence spanning residues 937 to 958 | TAGGAGAAGTGTTTCGACTAAC |
| 5 | Reverse Primer for 123 bp target sequence spanning residues 1038 to 1059 | CAAGAGCAATCATGGGATTATG |
| 6 | Probe for 123 bp target sequence spanning residues 1000 to 1024 (5'FAM - 3'BkFQ) | TTTACAATTGAGTTGAATTGTGTTCAAGC |
| 7 | Probe for 123 bp target sequence spanning residues 995 to 1020 (5'FAM - 3'BkFQ) | AAAAGAACACAATTCAACTCAATTGCTACG |
| 8 | Probe for 123 bp target sequence spanning residues 1000 to 1024 (5'FAM - 3'BkFQ) | CAATTGAGTTGAATTGTGTTCAAGC |
| 9 | Probe for 123 bp target sequence spanning residues 995 to 1020 | GAACACAATTCAACTCAATTGCTACG |

Based on a publicly available software (e.g., BLAST), SEQ ID NO:1 is conserved (e.g., 100% sequence identity) in 672 *Salmonella* Typhimurium strains listed by Genbank accession number in Table 2.

**Table 2: Strains of Salmonella Typhimurium in which SEQ ID NO:1 is 100% Conserved**

| | | | |
|---|---|---|---|
| LVHC01000004.1 | LAPF01000001.1 | CTPI01000002.1 | CTHF01000003.1 |
| LVHA01000006.1 | LAPD01000063.1 | CTPH01000002.1 | CTHE01000002.1 |
| LVGZ01000004.1 | LAPC01000018.1 | CTPG01000002.1 | CTHD01000002.1 |
| LVGY01000004.1 | LAOX01000013.1 | CTPF01000002.1 | CTHC01000003.1 |
| LVGX01000005.1 | JZAH01000029.1 | CTPE01000003.1 | CTHB01000001.1 |
| LVGW01000004.1 | JZAB01000025.1 | CTPD01000001.1 | CTHA01000002.1 |
| LVGU01000047.1 | JYZR01000005.1 | CTPC01000003.1 | CTGZ01000002.1 |
| LVGT01000051.1 | JYZC01000010.1 | CTPB01000003.1 | CTGY01000002.1 |
| LVGS01000046.1 | JYZB01000030.1 | CTPA01000004.1 | CTGX01000005.1 |
| LVGR01000047.1 | JYYZ01000004.1 | CTOZ01000002.1 | CTGW01000025.1 |
| LVGQ01000013.1 | JYYU01000025.1 | CTOY01000002.1 | CTGV01000002.1 |
| LVGP01000004.1 | JYYT01000016.1 | CTOX01000003.1 | CTGU01000006.1 |
| LVGN01000006.1 | JYYQ01000066.1 | CTOW01000001.1 | CTGT01000002.1 |
| LVGM01000008.1 | JYYL01000011.1 | CTOV01000003.1 | CTGS01000002.1 |
| LVGL01000007.1 | JYYK01000018.1 | CTOU01000002.1 | CTGR01000004.1 |
| LVGK01000003.1 | JYYH01000035.1 | CTOT01000003.1 | CTGQ01000002.1 |
| LVGJ01000009.1 | JYYC01000006.1 | CTOS01000002.1 | CTGP01000004.1 |
| LVGI01000006.1 | JYYB01000003.1 | CTOR01000003.1 | CTGO01000001.1 |
| LVGH01000005.1 | JYYA01000024.1 | CTOQ01000004.1 | CTGN01000001.1 |
| LVGG01000006.1 | JYXZ01000019.1 | CTOP01000003.1 | CTGM01000002.1 |
| LVGF01000006.1 | JYXX01000004.1 | CTOO01000002.1 | CTGL01000004.2 |
| LVFW01000011.1 | JYXW01000003.1 | CTON01000002.1 | CTGK01000002.1 |
| LVFV01000011.1 | JYXT01000019.1 | CTOM01000004.1 | CTGJ01000035.1 |
| LVFT01000012.1 | JYXK01000009.1 | CTOL01000003.1 | CTGI01000004.1 |
| LVFS01000014.1 | JYXH01000002.1 | CTOK01000002.1 | CTGH01000004.1 |
| LVFR01000005.1 | JYXC01000008.1 | CTOJ01000001.1 | CTGG01000002.1 |
| LVFQ01000008.1 | JYWW01000034.1 | CTOI01000002.1 | CTGF01000002.1 |
| LVFP01000006.1 | JYWU01000014.1 | CTOH01000003.1 | CTGE01000002.1 |
| LVFO01000002.1 | JYWT01000009.1 | CTOG01000003.1 | CTGD01000002.1 |
| LVFN01000004.1 | JYWA01000003.1 | CTOF01000002.1 | CTGC01000002.1 |
| LVFM01000005.1 | JYVW01000007.1 | CTOE01000002.1 | CTGB01000004.1 |
| LVFL01000037.1 | JYVV01000004.1 | CTOD01000002.1 | CTGA01000002.1 |
| LVFK01000003.1 | JYVU01000035.1 | CTOC01000003.1 | CTFZ01000004.1 |
| LVFJ01000013.1 | JYVL01000001.1 | CTOB01000002.1 | CTFY01000002.1 |
| LVFI01000003.1 | JYVJ01000004.1 | CTOA01000004.1 | CTFX01000001.1 |
| LVFH01000010.1 | JYVG01000055.1 | CTNZ01000003.1 | CTFW01000003.1 |
| LUJG01000006.1 | JYVE01000037.1 | CTNY01000003.1 | CTFV01000004.1 |
| LUJF01000005.1 | JYUZ01000004.1 | CTNX01000002.1 | CTCJ01000002.1 |
| LUJE01000003.1 | JYUY01000010.1 | CTNW01000004.1 | CTCI01000003.1 |
| LUJC01000020.1 | JYUP01000020.1 | CTNV01000003.1 | CTCH01000003.1 |
| LUIZ01000005.1 | JYTX01000005.1 | CTNU01000002.1 | CTCG01000002.1 |
| LUIY01000005.1 | JYTW01000027.1 | CTNT01000002.1 | CTCF01000002.1 |
| LUIW01000007.1 | JYTU01000018.1 | CTNS01000003.2 | CTCE01000003.1 |
| LUIU01000061.1 | JYTT01000012.1 | CTNR01000001.1 | CTCD01000002.1 |
| UIT01000019.1 | JYTQ01000001.1 | CTNQ01000001.1 | CTCC01000044.1 |
| UIS01000029.1 | JYTP01000017.1 | CTNP01000002.1 | CTCB01000003.1 |
| LUIR01000027.1 | JYTL01000012.1 | CTNO01000002.1 | CTCA01000003.1 |
| LUIQ01000009.1 | JYTI01000010.1 | CTNN01000003.1 | CTBZ01000002.1 |
| LUIP01000019.1 | JYTD01000002.1 | CTNM01000002.1 | CTBY01000002.1 |
| LUIO01000023.1 | JYTB01000004.1 | CTNL01000002.1 | CTBX01000003.1 |
| LUIN01000035.1 | JYSY01000001.1 | CTNK01000004.1 | CTBW01000004.1 |
| LUIM01000025.1 | JYSX01000031.1 | CTNJ01000001.1 | CTBV01000003.1 |
| LUIL01000040.1 | JYSO01000094.1 | CTNI01000002.1 | CTBU01000002.1 |
| LUIK01000041.1 | JYSN01000051.1 | CTNH01000002.1 | CTBT01000001.1 |
| LUIJ01000024.1 | JYSM01000007.1 | CTNG01000036.1 | CTBS01000003.1 |
| LUII01000036. 1 | JYSL01000029.1 | CTNF01000003.1 | CTBR01000004.1 |
| LUIH01000014.1 | JYSG01000016.1 | CTNE01000002.1 | CTBQ01000003.1 |
| LUIG01000040.1 | JYSB01000024.1 | CTND01000002.1 | CTBP01000005.1 |
| LUIF01000036.1 | JYSA01000003.1 | CTNC01000001.1 | CTBO01000002.1 |
| LUIE01000017.1 | JYRX01000002.1 | CTNB01000046.1 | CTBN01000003.1 |
| LUID01000024.1 | JYRW01000014.1 | CTNA01000004.1 | CTBM01000003.1 |
| LUIC01000116.1 | JYRT01000085.1 | CTMZ01000002.1 | CTBK01000003.1 |
| LUIB01000040.1 | JYRQ01000034.1 | CTMY01000004.1 | CTBJ01000002.1 |
| LUIA01000027.1 | JYRM01000039.1 | CTMX01000002.1 | CTBI01000002.1 |
| LUHZ01000025.1 | JYRD01000017.1 | CTMW01000004.1 | CTBH01000003.1 |
| LUHY01000038.1 | JYRC01000016.1 | CTMV01000003.1 | CTBF01000004.1 |
| LUHX01000025.1 | JYQU01000020.1 | CTMU01000003.1 | CTBE01000004.1 |
| LUHV01000033.1 | JYQM01000015.1 | CTMT01000004.1 | CTBC01000002.1 |
| LUHU01000040.1 | JYQC01000008.1 | CTMS01000002.1 | CTBB01000002.1 |
| LUHT01000028.1 | JYQA01000030.1 | CTMR01000004.1 | CTBA01000002.1 |
| LUHS01000024.1 | JYPT01000005.1 | CTMQ01000003.1 | CTAZ01000003.1 |
| LUHR01000036.1 | JYPP01000050.1 | CTMP01000002.1 | CTAY01000001.1 |
| LONA01000003.1 | JYPO01000064.1 | CTMO01000003.1 | CTAX01000001.1 |
| LKJI01000024.1 | JYPM01000008.1 | CTMN01000003.1 | CTAW01000001.1 |
| LKJE01000032.1 | JUIT01000021.1 | CTMM01000003.1 | CTAV01000002.1 |
| LKJD01000006.1 | JRZW01000002.1 | CTML01000002.1 | CTAU01000001.1 |
| LJJK01000144.1 | JRZV01000001.1 | CTMK01000002.1 | CTAT01000003.1 |
| LIOJ010000O1.1 | JRZU01000002.1 | CTMJ01000003.1 | CTAS01000002.1 |
| LIOG01000016.1 | JRZT01000030.1 | CTMI01000002.1 | CTAR01000002.1 |
| LIOF01000021.1 | JRZS01000015.1 | CTMH01000003.1 | CTAQ01000002.1 |
| LIOB01000013.1 | JRZR01000010.1 | CTMG01000002.1 | CTAP01000002.1 |
| LINM01000O04.1 | JRZQ01000006.1 | CTMF01000002.1 | CTAO01000002.1 |
| LINL01000011.1 | JRZO01000062.1 | CTME01000001.1 | CTAN01000001.1 |
| LINK01000002.1 | JRZN01000054.1 | CTMD01000003.1 | CTAM01000004.1 |
| LINJ01000020.1 | JRZM01000003.1 | CTMC01000004.1 | CTAL01000003.1 |
| LINF01000009.1 | JRZL01000059.1 | CTMB01000002.1 | CTAK01000004.1 |
| LINE01000010.1 | JRZK01000010.1 | CTMA01000002.1 | CTAJ01000002.1 |
| LINB01000005.1 | SJRZJ01000012.1 | CTLZ01000002.1 | CTAI01000002.1 |
| LIMZ01000030.1 | SJRZI01000017.1 | CTLY01000002.1 | CTAH01000003.1 |
| LIMV01000008.1 | JRZH01000001.1 | CTLX01000004.1 | CTAG01000004.1 |
| LIMU01000022.1 | JRYU01000004.1 | CTLW01000002.1 | CTAF01000002.1 |
| LIMT01000027.1 | JRYT01000009.1 | CTLV01000004.1 | CTAE01000002.1 |
| LIMN01000012.1 | JRGW01000022.1 | CTLU01000001.1 | CTAD01000002.1 |
| LIMM01000010.1 | JRGV01000045.1 | CTLT01000004.1 | CTAC01000004.1 |
| LIMH01000001.1 | JRGU01000009.1 | CTLS01000004.1 | CTAB01000002.1 |
| LIDY01000003.1 | JRGT01000034.1 | CTLR01000003.1 | CQIE01000002.1 |
| LHOM01000023.1 | JRGS01000001.1 | CTLQ01000001.1 | CQHW01000002.1 |
| LHOJ01000006.1 | JRGR01000006.1 | CTLP01000003.1 | CQHV01000002.1 |
| LHOH01000004.1 | JHAI01000005.1 | CTLO01000001.1 | CQHU01000002.1 |
| LHOG01000003.1 | JHAH01000020.1 | CTLN01000003.1 | CQHS01000002.1 |
| LHOF01000033.1 | JHAG01000020.1 | CTLM01000004.1 | CQHR01000002.1 |
| LHNW01000001.1 | JHAF01000018.1 | CTLL01000004.1 | CQHQ01000002.1 |
| LHNS01000017.1 | JHAE01000013.1 | CTLK01000002.1 | CQHN01000002.1 |
| LHNM01000038.1 | FKJD01000009.1 | CTLJ01000002.1 | CQHJ01000001.1 |
| LHNL01000010.1 | FKJC01000004.1 | CTLI01000002.1 | CQHI01000002.1 |
| LHNI01000015.1 | FKJB01000003.1 | CTLH01000004.1 | CQHH01000002.1 |
| LHNH01000007.1 | CYID01000003.1 | CTLG01000004.1 | CQHG01000001.1 |
| LHNG01000002.1 | CYIC01000003.1 | CTLF01000002.1 | CQHF01000001.1 |
| LHMT01000026.1 | CYIB01000003.1 | CTLE01000002.1 | CQHE01000002.1 |
| LHMS01000025.1 | CYIA01000002.1 | CTLD01000004.1 | CQHD01000002.1 |
| LHMA01000007.1 | CYHZ01000003.1 | CTLC01000006.1 | CQHC01000002.1 |
| LHLX01000009.1 | CYHY01000003.1 | CTLB01000002.1 | CQHB01000002.1 |
| LHLU01000004.1 | CYHX01000003.1 | CTLA01000002.1 | CQHA01000001.1 |
| LHLP01000001.1 | CYHW01000002.1 | CTKZ01000002.1 | CQGZ01000001.1 |
| LHLO01000016.1 | CYHV01000002.1 | CTKI01000002.1 | CQGY01000002.1 |
| LHLK01000019.1 | CYHU01000003.1 | CTKG01000001.1 | CQGX01000002.1 |
| LHLF01000020.1 | CYHT01000003.1 | CTKD01000002.1 | CQGW01000002.1 |
| LHLA01000001.1 | CVMK01000002.1 | CTKC01000002.1 | CQGV01000002.1 |
| LHKL01000042.1 | CVMH01000002.1 | CTKB01000004.1 | CQGU01000002.1 |
| LHKK01000034.1 | CVKN01000003.1 | CTKA01000003.1 | CQGT01000001.1 |
| LHKD01000036.1 | CVKM01000005.1 | CTJZ01000002.1 | CQGS01000002.1 |
| LHKC01000003.1 | CVKK01000004.1 | CTJY01000002.1 | CQGR01000002.1 |
| LHKA01000038.1 | CVKJ01000005.1 | CTJX01000003.1 | CQGQ01000002.1 |
| LHJU01000003.1 | CVKH01000004.1 | CTJW01000002.1 | CQGP01000002.1 |
| LHJJ01000028.1 | CVKC01000002.1 | CTJV01000003.1 | CQGO01000002.1 |
| LHJF01000017.1 | CVKA01000004.1 | CTJU01000005.1 | CQGN01000002.1 |
| LHIX01000004.1 | CVJW01000001.1 | CTJT01000002.1 | CQGM01000002.1 |
| LHIW01000017.1 | CTQZ01000003.1 | CTJS01000002.1 | CQGL01000002.1 |
| LHIR01000013.1 | CTQY01000002.1 | CTJR01000003.1 | CQGK01000001.1 |
| LHIQ01000047.1 | CTQX01000004.1 | CTJQ01000002.1 | CQGJ01000002.1 |
| LHIK01000014.1 | CTQU01000002.1 | CTJP01000002.1 | CQGI01000001.1 |
| LHIE01000015.1 | CTQT01000003.1 | CTJO01000004.2 | CQGH01000002.1 |
| LHID01000040.1 | CTQS01000002.1 | CTJN01000001.1 | CQGG01000002.1 |
| LHHK01000011.1 | CTQR01000002.1 | CTJM01000004.1 | CIJW01000002.1 |
| LHHB01000001.1 | CTQQ01000001.1 | CTJK01000003.1 | CIJD01000002.1 |
| LHGZ01000002.1 | CTQP01000002.1 | CTJJ01000002.1 | CHJY01000001.1 |
| LHGY01000001.1 | CTQO01000002.1 | CTIL01000002.1 | CGGH01000001.1 |
| LHGL01000047.1 | CTQN01000002.1 | CTIK01000003.1 | CGDA01000002.1 |
| LHGJ01000005.1 | CTQM01000002.1 | CTIJ01000003.1 | CGCS01000001.1 |
| LHGI01000006.1 | CTQL01000098.1 | CTII01000002.1 | CGCQ01000002.1 |
| LHGH01000003.1 | CTQK01000004.1 | CTIH01000002.1 | CGCH01000002.1 |
| LHFW01000031.1 | CTQJ01000002.1 | CTIG01000002.1 | CFOA01000002.1 |
| LHFV01000044.1 | CTQI01000001.1 | CTIF01000004.1 | CFNX01000002.1 |
| LHFU01000031.1 | CTQH01000001.1 | CTIE01000003.1 | CFLD01000002.1 |
| LHFT01000032.1 | CTQG01000002.1 | CTID01000002.1 | BAKU01000002.1 |
| LHFS01000022.1 | CTQF01000003.1 | CTIC01000002.1 | AYVJ01000114.1 |
| LHFP01000043.1 | CTQE01000004.1 | CTIB01000003.1 | AYUQ01000108.1 |
| LHFF01000013.1 | CTQD01000002.1 | CTIA01000004.1 | AUXR01000008.1 |
| LHEY01000021.1 | CTQC01000001.1 | CTHZ01000002.1 | AUXE01000003.1 |
| LHEX01000008.1 | CTQB01000003.1 | CTHY01000001.1 | AUVE01000003.1 |
| LHEW01000017.1 | CTQA01000002.1 | CTHX01000001.1 | AUVD01000043.1 |
| LHEV01000005.1 | CTPZ01000003.1 | CTHW01000001.1 | AUQU01000045.1 |
| LHER01000004.1 | CTPY01000003.1 | CTHV01000002.1 | AUQT01000034.1 |
| LHEQ01000025.1 | CTPX01000004.1 | CTHU01000002.1 | AUQO01000062.1 |
| LHEP01000049.1 | CTPW01000002.1 | CTHT01000004.1 | AUQN01000039.1 |
| LHEA01000005.1 | CTPV01000002.1 | CTHS01000002.1 | AUQM01000038.1 |
| LFGM01000013.1 | CTPU01000005.1 | CTHR01000002.1 | AOXO01000096.1 |
| LFDY01000028.1 | CTPT01000001.1 | CTHQ01000002.1 | AOXD01000065.1 |
| LFDW01000010.1 | CTPS01000002.1 | CTHP01000002.1 | AOXC01000083.1 |
| LFCC01000059.1 | CTPR01000003.1 | CTHO01000004.1 | AJTU01000007.1 |
| LDPA01000008.1 | CTPQ01000002.1 | CTHN01000002.1 | AHVA01000018.1 |
| LAPQ01000066.1 | CTPP01000002.1 | CTHM01000002.1 | AHUZ01000018.1 |
| LAPP01000078.1 | CTPO01000002.1 | CTHL01000002.1 | AHUV01000085.1 |
| LAPO01000058.1 | CTPN01000002.1 | CTHK01000002.1 | AHUT01000031.1 |
| LAPN01000091.1 | CTPM01000005.1 | CTHJ01000001.1 | AHUS01000047.1 |
| LAPM01000076.1 | CTPL01000004.1 | CTHI01000039.1 | AERV01000023.1 |
| LAPH01000042.1 | CTPK01000003.1 | CTHH01000002.1 | ABAO01000006.1 |
| LAPG01000091.1 | CTPJ01000002.1 | CTHG01000002.1 | LUIV01000062.1 |

Also based on a publicly available software (e.g., BLAST), SEQ ID NO:1 is partially conserved (e.g., at least 99% sequence identity) in 48 *Salmonella* Typhimurium strains listed by Genbank accession number in Table 3.

**Table 3: Strains of Salmonella Typhimurium in which SEQ ID NO:1 is Partially Conserved**

| | | | |
|---|---|---|---|
| LVGO01000006.1 | LUJA01000002.1 | LDYH01000006.1 | AMEA02000013.1 |
| LVGE01000003.1 | LUIX01000003.1 | JYPX01000009.1 | AMDZ02000027.1 |
| LVGD01000004.1 | LFGR01000011.1 | JTED01000004.1 | AMDY01000216.1 |
| LVGC01000003.1 | LFGQ01000013.1 | AUQV01000023.1 | AMDX02000108.1 |
| LVGB01000001.1 | LFGP01000017.1 | ATWR01000084.1 | JRZX01000002.1 |
| LVGA01000003.1 | LFGN01000015.1 | AMEH02000182.1 | JRZP01000003.1 |
| LVFZ01000005.1 | LFDX01000009.1 | AMEG02000055.1 | AUQL01000040.1 |
| LVFY01000001.1 | LFCI01000010.1 | AMEF02000044.1 | LFGO01000009.1 |
| LVFX01000003.1 | LFCH01000028.1 | AMEE02000031.1 | LFDZ01000012.1 |
| LVFU01000005.1 | LFCG01000008.1 | AMED02000059.1 | LECA01000009.1 |
| LUJD01000002.1 | LECD01000013.1 | AMEC02000055.1 | LUHW01000058.1 |
| LUJB01000005.1 | LECC01000010.1 | AMEB02000105.1 | LECB01000012.1 |

Based on a publicly available software (e.g., BLAST), SEQ ID NO:1 is not found in the following non-Typhimurium *Salmonella* strains: Salmonella_enterica_serovar_Ohio_CFSAN001079 Salmonella_enterica_serovar_Newport_WA14881 Salmonella_enterica_serovar_Schwarzengrund_SL480 Salmonella_enterica_serovar_Gallinarum_28791 Salmonella_enterica_serovar_Thompson_2010K1863 Salmonella_enterica_serovar _Kentucky_CDC191 Salmonella_enterica_serovar_Typhi_BL196 Salmonella_enterica_serovar_Enteritidis_P125109 Salmonella_enterica_serovar_Agona_72A52 Salmonella_enterica_serovar_Saintpaul_JO2008 Salmonella_enterica_serovar_Heidelberg_SL476 Salmonella_enterica_serovar_Virchow_ATCC51955 Salmonella_enterica_serovar_Paratyphi_B_SARA62 Salmonella_enterica_serovar_MontevideoMDA09249507 Salmonella_enterica_serovar_Muenchen_2009K0951 Salmonella_enterica_serovar_Bareilly_CFSAN000197 Salmonella_enterica_serovar_Virchow_SL491 Salmonella_enterica_serovar_Pullorum_19945 Salmonella_enterica_serovar_Dublin_SL1438 Salmonella_enterica_serovar_Stanley_060538

Further described is a detection method is based on the identification of residues 749-1697 of *Salmonella* Typhimurium ACCESSION CP007235 REGION: 658819...662133 (see SEQ ID NO:2 in Table 1). The detection method of the invention is based on the identification of residues 755-1063 of *Salmonella* Typhimurium ACCESSION CP007235 REGION: 658819...662133 (see SEQ ID NO:3 in Table 1). In some embodiments, the detection method of the incorporates unlabeled primers and labeled probes for the detection of *Salmonella* Typhimurium.

### Oligonucleotides

Oligonucleotides for the method of the instant invention are set forth in SEQ ID NOs: 4-9.

Disclosed oligonucleotides can be used as primers for PCR amplification and as hybridization probes. Primers and probes are shown in Table 1.

The nucleic acid probes can contain a detectable label. In some embodiments, the probe comprises a reporter-quencher combination as employed in a double-stranded probe, a TAQMAN™ probe, a molecular beacon probe, a SCORPION™ probe, a dual hybridization probe, or an ECLIPSE™ probe. In some embodiments, a double-stranded probe comprises two completely or partially complementary strands. In some embodiments, one strand of the double-stranded probe comprises a reporter on the 5' end and the other strand comprises a quencher on the 3' end such that when the two strands hybridize, the reporter and quencher face each other and the quencher quenches the fluorescence emitted by the reporter. During PCR, the strands separate, allowing the reporter to fluoresce and to be detected. In some embodiments, each strand of the double-stranded probe includes a reporter at one end (e.g., the 5' end) and a quencher at the other end (e.g., the 3' end). When the two strands hybridize with each other, the reporter from the first strand is in close proximity with the quencher of the second strand such that fluorescence quenching occurs. During PCR, the strands separate, allowing the reporter to fluoresce and to be detected. In an embodiment, the probe is a double-stranded probe as described in US Patent 9,194,007. In an embodiment, a reporter-quencher pair used in a double-stranded probe is 6-FAM and Iowa Black® FQ.

### III. Methods

The oligonucleotides can be used in a method for selectively detecting the presence of *Salmonella* Typhimurium in a sample.

In the method of the invention, the reaction mixture comprises a primer pair for amplification of residues 755-1063 of SEQ ID NO:3. In some embodiments, the reaction mixture comprises a primer pair for amplification of a sequence 95%, 97%, or 99% homologous to SEQ ID NO:3. In some embodiments, the reaction mixture comprises a primer pair for amplification of a sequence of SEQ ID NO:3 or a portion thereof. In some embodiments, the primer pair for amplification of the nucleic acid region of SEQ ID NO:3 comprises SEQ ID NO:4 and SEQ ID NO:5.

In some embodiments, the method further comprises a probe for the nucleic acid region to be detected. In certain embodiments, the probe comprises a detectable label. In some embodiments, the probe is a single-stranded probe comprising SEQ ID NO:6, SEQ ID NO:7, SEQ ID NO:8, or SEQ ID NO:9. In some embodiments, each probe is labeled with a reporter on one end (e.g., the 5' end) and a quencher on the other end (e.g., the 3' end). In some embodiments, the probe is a double-stranded probe comprising SEQ ID NO:6 and SEQ ID NO:7 (e.g., SEQ ID NO:6 can hybridize to SEQ ID NO:7) with each strand having a reporter on one end (e.g., the 5' end) and a quencher on the other end (e.g., the 3' end).

The next step of the method comprises performing PCR amplification (e.g., real-time PCR) of the nucleic acids of the sample using the reaction mixture. In some embodiments, PCR amplification is performed in partitions (e.g, droplets). Methods and compositions for partitioning a solution are described, for example, in WO 2012/135259, WO 2014/117088, WO 2010/036352, and US Patent 9,156,010.

In the last step of the method, the presence of *Salmonella* Typhimurium is selectively detected by detecting the amplified nucleic acids. In some embodiments, the detecting step comprises sequencing the amplified nucleic acids.

### IV. Kits

In another aspect, kits for detecting *Salmonella* Typhimurium in a sample according to the methods described herein are provided. In some embodiments, a kit comprises a primer pair as described herein. In some embodiments, the kit further comprises probes as described herein. In some embodiments, the kit further comprises assay components including a lysis reagent, a DNA polymerase, dNTPs, a buffer, a negative control, and a positive control. In some embodiments, the kit further comprises instructions for carrying out the methods described herein.

### V. Examples

### Example 1 - Comparison of Salmonella Typhimurium assay of the instant invention to Salmonella spp. assay

In this example, the *Salmonella* Typhimurium assay of the instant invention was compared to a commercially available *Salmonella* spp. Assay. In the experiment, eleven Salmonella serovars that are most relevant for the food industry were tested with the *Salmonella* Typhimurium assay of this disclosure and with the iQ-Check Salmonella spp. II Assay (Bio-Rad). The eleven *Salmonella* strains were streaked on a TCS Petri dish and allowed to grow for 24 hr at 37°C. Individual colonies were then picked, diluted in 500µL sterile water and 5µL were tested with each assay using the Bio-Rad CFX96 Touch™ Real-Time PCR Detection System. For the *Salmonella* Typhimurium assay, the double-stranded probe comprised SEQ ID NOs 6 and 7. Each strand of the double-stranded probe was labeled with 6-FAM on the 5' end and Iowa Black™ FQ on the 3'end. The double-stranded probe was synthesized by Integrated DNA Technologies using phosphoramidite chemistry. Results are shown in Table 4.

**Table 4: Comparison of Assays**

| | **Bio-Rad iQ-Check *Salmonella* spp. II assay** | | | ***Salmonella* Typhimurium assay** | | |
|---|---|---|---|---|---|---|
| *Serovars* | **Target Cq** | **Internal control Cq** | **Result** | **Target Cq** | **Internal control Cq** | **Result** |
| Negative ctrl | **N/A** | 32,84 | Negative | N/A | 32,49 | Negative |
| Positive ctrl | 31,72 | 32,24 | Positive | 31,95 | 31,78 | Positive |
| **Typhimurium** | **19,69** | **34,60** | **Positive** | **19,36** | **33,43** | **Positive** |
| **Monophasic Typhimurium** | **18,35** | **N/A** | **Positive** | **18,08** | **34,2** | **Positive** |
| Enteritidis | 20,34 | 32,72 | Positive | N/A | 32,15 | Negative |
| Infantis | 20,86 | 32,40 | Positive | N/A | 31,78 | Negative |
| Virchow | 19.71 | 33.28 | Positive | N/A | 32 | Negative |
| Hadar | 21,20 | 32,29 | Positive | N/A | 31,89 | Negative |
| Paratyphi B Java | 20,24 | 33,27 | Positive | N/A | 31,91 | Negative |
| Livingstone | 20,84 | 33,21 | Positive | N/A | 32,38 | Negative |
| Kentucky | 18,67 | 34,26 | Positive | N/A | 32,17 | Negative |
| Dublin | 21,24 | 32,68 | Positive | N/A | 31,99 | Negative |
| Newport | 20,23 | 32,67 | Positive | N/A | 31,98 | Negative |

The results shown in Table 4 illustrate that only typhimurium is detected by the *Salmonella* Typhimurium assay. The results also show that the sensitivities of both assays are identical and that the *Salmonella* Typhimurium assay can be used as a primary screening assay or as a confirmatory, serotyping assay.

### Example 2 - Assay Selectivity

This example illustrates assay selectivity of the instant invention. One-hundred and nine *Salmonella enterica subsp. enterica serovars* and *Salmonella enterica subspecies* (in italics in Table 4) were tested with the *Salmonella* Typhimurium assay. The same method and probes as in Example 1 were used in this experiment. The organisms tested are shown in Table 5.

**Table 5: Selectivity**

| | | | |
|---|---|---|---|
| Abaetetuba | Dublin | Kedougou | Quentin |
| Aberdeen | Emek | Lomita | Rostock |
| Adelaïde | Duisberg | Livingstone | Salamae |
| Agama | Enteritidis | Manica | Rubislaw |
| Albany | Fischerkietz | London | Senftenberg |
| Anatum | Ferruch | Miami | Saint Paul |
| *arizonae** | Give | Minnesota | Schwarzengrund |
| Bambylor | Gaminara | Maregrosso | Singapore |
| Bareilly | Gallinarum | Mbandaka | Sheffield |
| Berta | Glostrup | Muenchen | Sundsvall |
| Betioky | Grumpensis | Montevideo | Springs |
| Blegdam | Grabow | Moscow | Strasbourg |
| Blockley | Goldgoast | Napoli | Taksony |
| bongori | Havana | Nienstedten | Tallahassee |
| Braenderup | Hadar | Naestved | Tournai |
| Brandenburg | Guinea | Newport | Tenessee |
| Bredeney | Havanna | Nottingham | Thompson |
| Budapest | *houtenae** | Oranienburg | Treforest |
| California | Illinois | Ouakam | Tranoroa |
| Cerro | Heidelberg | Okatie | Utrecht |
| Carrau | Indiana | Ohio | Virchow |
| Canoga | *indica** | Phoenix | Zuerich |
| Crossness | Inverness | Panama | Yoruba |
| Cubana | Johannesburq | **Paratyphi B**** | Wayne |
| Choleraesuis | Infantis | Paratyphi B java | Worthington |
| *diarizonae** | Kentucky | Postdam | |
| Dalhem | Kirkee | Poona | |
| Derby | Kottbus | Puttin | |

Of the organisms listed in Table 5, all but Paratyphi B were not detected with the assay. The results illustrate that the *Salmonella* Typhimurium assay is highly selective for *Salmonella* Typhimurium.

### Example 3 - Assay Exclusivity

This example illustrates assay exclusivity of the instant invention. Thirty-nine non-Salmonella bacteria were tested with the *Salmonella* Typhimurium assay. The same method and probe as in Example 1 was used in this experiment. The bacteria tested are tabulated in Table 6. None of the bacteria listed in Table 6 were detected by the *Salmonella* Typhimurium assay, illustrating assay exclusivity.

**Table 6: Exclusivity**

| | | |
|---|---|---|
| Acinetobacter *baumanii* | *Enterobacter sakazakii* | *Pantoea agglomerans* |
| *Aeromonas hydrophila* | *Enterobacter aerogenes* | *Proteus mirabilis* |
| *Aeromonas hydrophila*/*caviae* | *Enterobacter asburiae* | *Pseudomonas fluorescens* |
| *Bacillus licheniformis* | *Enterobacter amnigenus* | *Pseudomonas aeruginosa* |
| *Bacillus cereus* | *Enterobacter cowanii* | *Raoultella terrigena* |
| *Campylobacter jejuni* | *Enterococcus faecium* | *Serratia marcescens* |
| *Campylobacter coli* | *Escherichia coli* | *Shigella flexneri* |
| *Campylobacter lari* | *Escherichia hermanii* | *Shigella sonnei* |
| *Campylobacter upsaliensis* | *Hafnia alvei* | *Staphylococcus aureus* |
| *Citrobacter freundii* | *Klebsiella oxytoca* | *Staphylococcus intemmedius* |
| *Cronobacter sakazakii* | *Klebsiella pneumoniae* | *Staphylococcus xylosus* |
| *Enterobacter cloacae* | *Listeria monocytogenes* | *Staphylococcus epidermidis* |
| *Enterobacter pyrinus* | *Micrococcus luteus* | *Yersinia enterocoloitica* |

### Example 4 - Assay Specificity

This example illustrates assay specificity of the instant invention. Seventy-nine *Salmonella* Typhimurium serovars were tested with the *Salmonella* Typhimurium assay. The same method and probe as in Example 1 was used in this experiment. The bacteria tested are tabulated in Table 7. All of the bacteria listed in Table 7 were detected by the *Salmonella* Typhimurium assay, illustrating assay specificity.

**Table 7: Specificity**

| **Serovar** | **Antigenic formula** | **Comment** | **Primary source** | **Origin** | **Strain number (Bio-Rad Library)** | **Strain number (Other Library)** |
|---|---|---|---|---|---|---|
| Typhimurium | 1,4,[5],12:i:1,2 | | Anses | Brine | 002 | n° Anses: 38.09 |
| Typhimurium | 1,4,[5],12:i:1,2 | | Anses | Beef meat | 003 | n° Anses: 442.09 |
| Typhimurium | 1,4,[5],12:i:1,2 | | Anses | Pork (crépine de porc) | 004 | n° Anses: 447.09 |
| Typhimurium | 1,4,[5],12:i:1,2 | | Anses | Lamb with sauce | 005 | n° Anses: 591.09 |
| Typhimurium | 1,4,[5],12:i:1,2 | | Anses | Stuffed quail | 006 | n° Anses: 695.09 |
| Typhimurium | 1,4,[5],12:i:1,2 | | Anses | Culture from lamb feces | 007 | n° Anses: 839.09 |
| Typhimurium | 1,4,[5],12:i:1,2 | | Anses | Environment (Duck) | 008 | n° Anses: 838.09 |
| Typhimurium | 1,4,[5],12:i:1,2 | | Anses | Culture from horse feces | 009 | n° Anses: 553.11 |
| Typhimurium | 1,4,[5],12:i:1,2 | | Anses | White pepper | 010 | n° Anses: 564.11 |
| Typhimurium | 1,4,[5],12:i:1,2 | | Anses | Hoki filet with cream | 011 | n° Anses: 708.11 |
| Typhimurium | 1,4,[5],12:i:1,2 | | Anses | Pigeon viscera | 012 | n° Anses: 781.11 |
| Typhimurium | 1,4,[5],12:i:1,2 | | Anses | Compost | 013 | n° Anses: 792.11 |
| Typhimurium | 1,4,[5],12:i:1,2 | | Anses | Environmental (Chicken) | 014 | n° Anses: 835.11 |
| Typhimurium | 1,4,[5],12:i:1,2 | | Anses | Streaky ham | 015 | n° Anses: 840.11 |
| Typhimurium | 1,4,[5],12:i:1,2 | | Anses | Whole quail | 016 | n° Anses: 845.11 |
| Typhimurium | 1,4,[5],12:i:1,2 | | Anses | Spareribs | 017 | n° Anses: 880.11 |
| Typhimurium | 1,4,[5],12:i:1,2 | | Anses | Culture from swine feces | 018 | n° Anses: 886.11 |
| Typhimurium | 1,4,[5],12:i:1,2 | | Anses | Raw milk (cow) | 019 | n° Anses: 907.11 |
| Typhimurium | 1,4,[5],12:i:1,2 | | Anses | Sausage | 020 | n° Anses: 976.11 |
| Typhimurium | 1,4,[5],12:i:1,2 | | Anses | Tomato filling | 021 | n° Anses: 977.11 |
| Typhimurium | 1,4,[5],12:i:1,2 | | Anses | Stuffed potatoes | 022 | n° Anses: 979.11 |
| Typhimurium | 1,4,[5],12:i:1,2 | | Anses | Fish meal | 023 | n° Anses: 985.11 |
| Typhimurium | 1,4,[5],12:i:1,2 | | Anses | Pet food | 043 | n° Anses: 1175.11 |
| Typhimurium | 1,4,[5],12:i:1,2 | | Anses | Foie gras (Liver) | 030 | n° Anses: 119.11 |
| Typhimurium | 1,4,[5],12:i:1,2 | | ADRIA Development | Milk powder | 081 | ADRIA n°4 |
| Typhimurium | 1,4,[5],12:i:1,2 | | ADRIA Development | Pasteurized liquid egg | 082 | ADRIA n°13 |
| Typhimurium | 1,4,[5],12:i:1,2 | | ADRIA Development | Pasteurized liquid egg | 083 | ADRIA n°206 |
| Typhimurium | 1,4,[5],12:i:1,2 | | ADRIA Development | Egg yolk | 084 | ADRIA n°472 |
| Typhimurium | 1,4,[5],12:i:1,2 | | ADRIA Development | Pasteurized liquid egg | 085 | ADRIA n°776 |
| Typhimurium | 1,4,[5],12:i:1,2 | | ADRIA Development | Ready-to-eat | 086 | CIP 58.58 |
| Typhimurium | 1,4,[5],12:i:1,2 | | ADRIA Development | Foie (Liver) | 087 | ADRIA n°19 |
| Typhimurium | 1,4,[5],12:i:1,2 | | ADRIA Development | Raw ground meat | 088 | ADRIA n°22 |
| Typhimurium | 1,4,[5],12:i:1,2 | | ADRIA Development | Ready-to-eat | 089 | ADRIA n°167 |
| Typhimurium | 1,4,[5],12:i:1,2 | | ADRIA Development | Chipolatas sausages | 090 | ADRIA n°193 |
| Typhimurium | 1,4,[5],12:i:1,2 | | ADRIA Development | Chipolatas sausages | 091 | ADRIA n°830 |
| Typhimurium | 1,4,[5],12:i:1,2 | | ADRIA Development | Merguez sausages | 092 | ADRIA n°911 |
| Typhimurium | 1,4,[5],12:i:1,2 | | ADRIA Development | Chipolatas sausages | 093 | ADRIA n°987 |
| Typhimurium | 1,4,[5],12:i:1,2 | | ADRIA Development | Meat (pâté) | 094 | ADRIA n°4874 |
| Typhimurium | 1,4,[5],12:i:1,2 | | ADRIA Development | Frozen meat | 095 | A00C003 |
| Typhimurium | 1,4,[5],12:i:1,2 | | ADRIA Development | Frozen meat | 096 | A00C004 |
| Typhimurium | 1,4,[5],12:i:1,2 | | ADRIA Development | Frozen beef trim | 097 | A00C059 |
| Typhimurium | 1,4,[5],12:i:1,2 | | ADRIA Development | ground beef | 098 | A00C060 |
| Typhimurium | 1,4,[5],12:i:1,2 | | ADRIA Development | Pork | 106 | Ad1070 |
| Typhimurium | 1,4,[5],12:i:1,2 | | ADRIA Development | Pork | 107 | ST325 |
| Typhimurium | 1,4,[5],12:i:1,2 | | ADRIA Development | Pork | 108 | ST1 |
| Typhimurium | 1,4,[5],12:i:1,2 | | ADRIA Development | Pork | 109 | ST394 |
| Typhimurium | 1,4,[5],12:i:1,2 | | ADRIA Development | Pork | 110 | ST719 |
| Typhimurium | 1,4,[5],12:i:1,2 | | ADRIA Development | Pork | 111 | ST11 |
| Typhimurium | 1,4,[5],12:i:1,2 | | ADRIA Development | Liquid egg | 113 | JES411 |
| Typhimurium | 1,4,[5],12:i:1,2 | | ADRIA Development | Beef trim | 116 | Ad913 |
| Typhimurium | 1,4,[5],12:i:1,2 | | ADRIA Development | Pork | 118 | Ad1249 |
| Typhimurium | 1,4,[5],12:i:1,2 | | ADRIA Development | pork (crépine) | 119 | Ad1338 |
| Typhimurium | 1,4,[5],12:i:1,2 | | ADRIA Development | ground meat | 120 | Ad1410 |
| Typhimurium | 1,4,[5],12:i:1,2 | | ADRIA Development | Liquid egg | 121 | Ad1484 |
| Typhimurium | 1,4,[5],12:i:1,2 | | ADRIA Development | Drinking water from trough | 122 | Ad1546 |
| Typhimurium | 1,4,[5],12:i:1,2 | | ADRIA Development | salmon with vegetables | 123 | Ad1603 |
| Typhimurium | 1,4,[5],12:-:- | *non motile variant* | ADRIA Development | Tiramisu | 124 | Adl333 |
| Typhimurium | 1,4,[5],12:-:1,2 | *monophasic variant* | ADRIA Development | Hen | 125 | Ad1335 |
| Typhimurium | 1,4,[5],12:i:- | *monophasic variant* | ADRIA Development | Pork specialty | 126 | Ad1334 |
| Typhimurium | 1,4,[5],12:i:1,2 | | Anses | Environmental (Quail) | 160 | 2002LSAL00347 |
| Typhimurium | 1,4,[5],12:i:1,2 | | Anses | Environmental (goose) | 161 | 2016LSAL02607 |
| Typhimurium | 1,4,[5],12:i:- | *monophasic variant* | Anses | Environmental (Pork) | 162 | 2009LSAL04410 |
| Typhimurium | 1,4,[5],12:-:1,2 | *monophasic variant* | Anses | Environmental (Gallus gallushen) | 163 | 2010LSAL01759 |
| Typhimurium | 1,4,[5],1 2:i:- | *monophasic variant* | Anses | Environmental | 164 | 2011LSAL04681 |
| Typhimurium | 1,4,[5],12:i:- | *monophasic variant* | Anses | Environmental (Turkey) | 165 | 2012LSAL04635 |
| Typhimurium | 1,4,[5],12:i:1,2 | | Anses | Environmental (Gallus gallus) | 166 | 2013LSAL00987 |
| Typhimurium | 1,4,[5],12:i:- | *monophasic variant* | Anses | Environmental (Bovine) | 167 | 2014LSAL00857 |
| Typhimurium | 1,4,[5],12:i:- | *monophasic variant* | Anses | Pork meat | 168 | 2011LSAL06561 |
| Typhimurium | 1,4,[5],12:i:- | *monophasic variant* | Anses | Veal meat | 169 | 2012LSAL05317 |
| Typhimurium | 1,4,[5],12:i:- | *monophasic variant* | Anses | Turkey meat | 170 | 2014LSAL02635 |
| Typhimurium | 1,4,[5],12:i:- | *monophasic variant* | Anses | Gallus gallus meat | 171 | 2014LSAL03913 |
| Typhimurium | 1,4,[5],12:i:- | *monophasic variant* | Anses | Poultry Feed | 172 | 2011LSAL04983 |
| Typhimurium | 1,4,[5],12:i:- | *monophasic variant* | Anses | cattle feed | 173 | 2012LSAL03407 |
| Typhimurium | 1,4,[5],12:i:- | *monophasic variant* | Anses | animal blood products | 174 | 2012LSAL03874 |
| Typhimurium | 1,4,[5],12:i:- | *monophasic variant* | Anses | cattle feed | 175 | 2015LSAL00792 |
| Typhimurium | 1,4,[5],12:i:- | *monophasic variant* | Anses | beef meat (carcass) | 176 | 2013LSAL02030 |
| Typhimurium | 1,4,[5],12:i:- | *monophasic variant* | Anses | pork (carcass) | 177 | 2015LSAL01461 |
| Typhimurium | 1,4,[5],12:i:- | *monophasic variant* | Anses | turkey (carcass) | 178 | 2013LSAL03886 |
| Typhimurium | 1,4,[5],12:i:- | *monophasic variant* | Anses | chicken (carcass) | 179 | 2016LSAL00194 |

## Claims

1. A method of selectively detecting the presence of *Salmonella* Typhimurium in a sample, the method comprising:
(a) providing a reaction mixture comprising a suitable primer pair for amplification of a sequence having at least 95% sequence identity over the entire length of the sequence of SEQ ID NO:3;
(b) performing PCR amplification of the nucleic acids of the sample using the reaction mixture of step (a) to amplify the sequence; and
(c) selectively detecting the presence of *Salmonella* Typhimurium by detecting the amplified sequence which has at least 95% sequence identity over the entire length of the sequence of SEQ ID No: 3.

2. The method of claim 1, wherein the reaction mixture comprises a primer pair for amplification of a sequence having at least 97% sequence identity over the entire length of the sequence of SEQ ID NO:3.

3. The method of claim 1, wherein the reaction mixture comprises a primer pair for amplification of a sequence having at least 99% sequence identity over the entire length of the sequence of SEQ ID NO:3.

4. The method of claim 1, wherein the reaction mixture comprises a primer pair for amplification of a sequence of SEQ ID NO:3, preferably the primer pair for amplification of the nucleic acid region of SEQ ID NO:3 comprises SEQ ID NO:4 and SEQ ID NO:5.

5. The method of any of claims 1 to 4, wherein the reaction mixture further comprises a probe for the nucleic acid region to be detected, preferably
(i) the probe comprises a detectable label; and/or
(ii) the probe comprises SEQ ID NO:6, SEQ ID NO:7, SEQ ID NO:8, or SEQ ID NO:9; and/or
(iii) the probe is selected from the group consisting of a double-stranded probe, a TAQMAN™probe, a molecular beacon probe, a dual hybridization probe, an ECLIPSE™ probe, and a SCORPION™ probe.

6. The method of claim 1, wherein the detecting the presence of *Salmonella* Typhimurium comprises sequencing the amplified nucleic acids.

7. The method of claim 1, wherein step (b) is performed in partitions.

8. A primer pair comprising the polynucleotide sequences as set forth in SEQ ID NO:4 and SEQ ID NO:5.

9. An isolated polynucleotide consisting of a polynucleotide sequence having at least 95% sequence identity, based on the BLASTN method of alignment, over the entire length of the polynucleotide sequence set forth in SEQ ID NO: 3.

10. The isolated polynucleotide of claim 9, wherein the isolated polynucleotide has at least 97% or at least 99% sequence identity over the entire length of the polynucleotide sequence set forth in SEQ ID NO:3.

11. The isolated polynucleotide of claim 9, wherein the isolated polynucleotide consists of
the polynucleotide sequence set forth in SEQ ID NO:3.

12. A kit for the detection of *Salmonella* Typhimurium in a sample, the kit comprising a primer pair comprising SEQ ID NO:4 and SEQ ID NO:5.

13. The kit of claim 12, further comprising a probe comprising SEQ ID NO:6 and SEQ ID NO:7.

14. The kit of claim 12, further comprising a probe comprising SEQ ID NO:6, SEQ ID NO:7, SEQ ID NO:8, or SEQ ID NO:9.

15. The kit of claim 13 or 14, further comprising at least one component selected from a lysis reagent, a DNA polymerase, dNTPs, a buffer, a negative control, a positive control, and instructions for performing a method to detect the presence of *Salmonella* Typhimurium in a nucleic acid sample.

## Patentansprüche

1. Verfahren zum selektiven Nachweis der Anwesenheit von *Salmonella typhimurium* in einer Probe, wobei das Verfahren umfasst:
(a) Bereitstellen eines Reaktionsgemischs, das ein geeignetes Primerpaar für die Amplifikation einer Sequenz, die wenigstens 95% Sequenzidentität über die gesamte Länge der Sequenz von SEQ ID Nr. 3 aufweist, umfasst;
(b) Durchführen einer PCR-Amplifikation der Nucleinsäuren der Probe unter Verwendung des Reaktionsgemischs von Schritt (a), um die Sequenz zu amplifizieren; und
(c) selektives Nachweisen der Anwesenheit von *Salmonella typhimurium* durch Nachweisen der amplifizierten Sequenz, die wenigstens 95% Sequenzidentität über die gesamte Länge der Sequenz von SEQ ID Nr. 3 aufweist.

2. Verfahren gemäß Anspruch 1, wobei das Reaktionsgemisch ein Primerpaar für die Amplifikation einer Sequenz, die wenigstens 97% Sequenzidentität über die gesamte Länge der Sequenz von SEQ ID Nr. 3 aufweist, umfasst.

3. Verfahren gemäß Anspruch 1, wobei das Reaktionsgemisch ein Primerpaar für die Amplifikation einer Sequenz, die wenigstens 99% Sequenzidentität über die gesamte Länge der Sequenz von SEQ ID Nr. 3 aufweist, umfasst.

4. Verfahren gemäß Anspruch 1, wobei das Reaktionsgemisch ein Primerpaar für die Amplifikation einer Sequenz von SEQ ID Nr. 3 umfasst, wobei das Primerpaar für die Amplifikation des Nucleinsäurebereichs von SEQ ID Nr. 3 vorzugsweise SEQ ID Nr. 4 und SEQ ID Nr. 5 umfasst.

5. Verfahren gemäß einem der Ansprüche 1 bis 4, wobei das Reaktionsgemisch weiterhin eine Sonde für den nachzuweisenden Nucleinsäurebereich umfasst, wobei vorzugsweise
(i) die Sonde einen nachweisbaren Marker umfasst; und/oder
(ii) die Sonde SEQ ID Nr. 6, SEQ ID Nr. 7, SEQ ID Nr. 8 oder SEQ ID Nr. 9 umfasst; und/oder
(iii) die Sonde aus der Gruppe ausgewählt ist, die aus einer doppelsträngigen Sonde, einer TAQMAN™-Sonde, einer Molecular-Beacon-Sonde, einer dualen Hybridisierungssonde, einer ECLIPSE™-Sonde und einer SCORPION™-Sonde besteht.

6. Verfahren gemäß Anspruch 1, wobei das Nachweisen der Anwesenheit von *Salmonella typhimurium* das Sequenzieren der amplifizierten Nucleinsäuren umfasst.

7. Verfahren gemäß Anspruch 1, wobei Schritt (b) in Partitionen durchgeführt wird.

8. Primerpaar, das die Polynucleotidsequenzen umfasst, die in SEQ ID Nr. 4 und SEQ ID Nr. 5 dargelegt sind.

9. Isoliertes Polynucleotid, das aus einer Polynucleotidsequenz besteht, die wenigstens 95% Sequenzidentität, bezogen auf die BLASTN-Alignment-Methode, über die gesamte Länge der in SEQ ID Nr. 3 dargelegten Polynucleotidsequenz aufweist.

10. Isoliertes Polynucleotid gemäß Anspruch 9, wobei das isolierte Polynucleotid wenigstens 97% oder wenigstens 99% Sequenzidentität über die gesamte Länge der in SEQ ID Nr. 3 dargelegten Polynucleotidsequenz aufweist.

11. Isoliertes Polynucleotid gemäß Anspruch 9, wobei das isolierte Polynucleotid aus der in SEQ ID Nr. 3 dargelegten Polynucleotidsequenz besteht.

12. Kit zum Nachweis von *Salmonella typhimurium* in einer Probe, wobei der Kit ein Primerpaar umfasst, das SEQ ID Nr. 4 und SEQ ID Nr. 5 umfasst.

13. Kit gemäß Anspruch 12, weiterhin umfassend eine Sonde, die SEQ ID Nr. 6 und SEQ ID Nr. 7 umfasst.

14. Kit gemäß Anspruch 12, weiterhin umfassend eine Sonde, die SEQ ID Nr. 6, SEQ ID Nr. 7, SEQ ID Nr. 8 oder SEQ ID Nr. 9 umfasst.

15. Kit gemäß Anspruch 13 oder 14, weiterhin umfassend wenigstens eine Komponente, die aus einem Lysereagens, einer DNA-Polymerase, dNTPs, einem Puffer, einer negativen Kontrolle, einer positiven Kontrolle und Anweisungen zum Durchführen eines Verfahrens zum Nachweis der Anwesenheit von *Salmonella typhimurium* in einer Nucleinsäureprobe ausgewählt ist.

## Revendications

1. Procédé permettant de détecter de manière sélective la présence de *Salmonella* Typhimurium dans un échantillon, ledit procédé comprenant :
(a) l'obtention d'un mélange réactionnel comprenant une paire d'amorces appropriées pour l'amplification d'une séquence ayant une identité de séquence d'au moins 95% sur toute la longueur de la séquence de SEQ ID NO:3 ;
(b) la réalisation d'une amplification par PCR des acides nucléiques de l'échantillon en utilisant le mélange réactionnel de l'étape (a) pour amplifier la séquence ; et
(c) la détection de manière sélective de la présence de *Salmonella* Typhimurium grâce à la détection de la séquence amplifiée qui a une identité de séquence d'au moins 95% sur toute la longueur de la séquence de SEQ ID NO:3.

2. Procédé selon la revendication 1, dans lequel le mélange réactionnel comprend une paire d'amorces pour l'amplification d'une séquence ayant une identité de séquence d'au moins 97% sur toute la longueur de la séquence de SEQ ID NO:3.

3. Procédé selon la revendication 1, dans lequel le mélange réactionnel comprend une paire d'amorces pour l'amplification d'une séquence ayant une identité de séquence d'au moins 99% sur toute la longueur de la séquence de SEQ ID NO:3.

4. Procédé selon la revendication 1, dans lequel le mélange réactionnel comprend une paire d'amorces pour l'amplification d'une séquence de SEQ ID NO:3, de préférence, la paire d'amorces pour l'amplification de la région d'acide nucléique de SEQ ID NO:3 comprend SEQ ID NO:4 et SEQ ID NO:5.

5. Procédé selon l'une quelconque des revendications 1 à 4, dans lequel le mélange réactionnel comprend en outre une sonde pour la région d'acide nucléique à détecter, de préférence,
(i) la sonde comprend un marqueur détectable ; et/ou
(ii) la sonde comprend SEQ ID NO:6, SEQ ID NO:7, SEQ ID NO:8 ou SEQ ID NO:9; et/ou
(iii) la sonde est choisie dans le groupe constitué par une sonde double-brin, une sonde TAQMAN™, une sonde balise moléculaire, une sonde d'hybridation double, une sonde ECLIPSE™ et une sonde SCORPION™.

6. Procédé selon la revendication 1, dans lequel la détection de la présence de *Salmonella* Typhimurium comprend le séquençage des acides nucléiques amplifiés.

7. Procédé selon la revendication 1, dans lequel l'étape (b) est réalisée dans des partitions.

8. Paire d'amorces comprenant les séquences polynucléotidiques telles que décrites dans SEQ NO:4 et SEQ ID NO:5.

9. Polynucléotide isolé constitué d'une séquence polynucléotidique ayant une identité de séquence d'au moins 95%, basée sur la méthode d'alignement BLASTN, sur toute la longueur de la séquence polynucléotidique décrite dans SEQ ID NO :3.

10. Polynucléotide isolé selon la revendication 9, ledit polynucléotide isolé ayant une identité de séquence d'au moins 97% ou d'au moins 99% sur toute la longueur de la séquence polynucléotidique décrite dans SEQ ID NO :3.

11. Polynucléotide isolé selon la revendication 9, ledit polynucléotide isolé étant constitué de la séquence polynucléotidique décrite dans SEQ ID NO :3.

12. Trousse pour la détection de *Salmonella* Typhimurium dans un échantillon, ladite trousse comprenant une paire d'amorces comprenant SEQ ID NO:4 et SEQ ID NO:5.

13. Trousse selon la revendication 12, comprenant en outre une sonde comprenant SEQ ID NO:6 et SEQ ID NO:7.

14. Trousse selon la revendication 12, comprenant en outre une sonde comprenant SEQ ID NO:6, SEQ ID NO:7, SEQ ID NO:8 et SEQ ID NO:9.

15. Trousse selon la revendication 13 ou 14, comprenant en outre au moins un composant choisi parmi un réactif de lyse, une ADN-polymérase, des dNTP, un tampon, un contrôle négatif, un contrôle positif, et des instructions pour réaliser un procédé permettant de détecter la présence de *Salmonella* Typhimurium dans un échantillon d'acide nucléique.
